# EUROPÄISCHE PATENTSCHRIFT

(11) **EP 0 546 032 B1**
(45) Veröffentlichungstag und Bekanntmachung des Hinweises auf die Patenterteilung: **20.03.1996**
(21) Anmeldenummer: 91915666.1
(22) Anmeldetag: 27.08.1991
(51) Int. Cl.: C12Q 1/00, G01N 33/543, C12N 11/04

(54) **IMMOBILISIERUNG VON ORGANISCHEN MAKROMOLEKÜLEN ODER BIOPOLYMEREN IN EINER POLYMERMEMBRAN**
IMMOBILIZATION OF ORGANIC MACROMOLECULES OR BIOPOLYMERS IN A POLYMER MEMBRANE
IMMOBILISATION DE MACROMOLECULES OU DE BIOPOLYMERES ORGANIQUES DANS UNE MEMBRANE POLYMERE

(30) Priorität: 31.08.1990 DE 4027728
(43) Veröffentlichungstag der Anmeldung: 16.06.1993
(73) Patentinhaber: BAYER AG, D-51368 Leverkusen (DE)
(72) Erfinder: HAMPP, Norbert, D-8000 München 40 (DE); SCHOLZE, Juliane, D-8000 München 40 (DE); BRÄUCHLE, Christoph, D-8000 München 83 (DE)
(86) Internationale Anmeldenummer: EP9101628
(87) Internationale Veröffentlichungsnummer: WO9204465

(56) Entgegenhaltungen:
- EP-A- 0 075 215
- EP-A- 0 238 951
- WO-A-89/07139
- JP-A-52 143 281

## Beschreibung

Die Erfindung geht aus von einem Verfahren zur Immobilisierung von organischen Makromolekülen oder Biopolymeren, bei dem die zu immobilisierenden Makromoleküle oder Biopolymere mit einer wäßrigen, lösungsmittelfreien, nicht ionogenen Polymerdispersion vermischt werden und diese Mischung in Form eines zusammenhängenden Films auf ein Substrat aufgetragen wird, der anschließend getrocknet wird. Ein derartiges Verfahren ist z. B. aus JP-A-52 143 281 (Patent Abstracts of Japan, Vol. 2., No. 38, 14.03.1978; Section C, 4711 C 77) bekannt. Ferner wird in EP-A-75 215 ein Verfahren zur Herstellung eines Mikroorganismen enthaltenden Dispersionsfilmes beschrieben. Als Filmbildner werden dabei wäßrige, lösungsmittelfreie und nicht ionogene Kunststoffdispersionen verwendet, z. B. Homopolymere aus Vinylacetat. Das so hergestellte Substrat dient zum Nachweis von Mikroorganismen hemmenden Stoffen in einer flüssigen Probe.

Bei dem erfindungsgemäßen Verfahren handelt es sich insbesondere um die Immobilisierung von Enzymen, Antikörpern, Antigenen, DNA/RNA und von Mikroorganismen, z. B. Bakterienzellen in einem Polymerfilm bzw. einer Polymermembran. Derartige Membranen sind ein wichtiger Bestandteil von Transducern für Biosensoren und besitzen chemisch-selektive Eigenschaften. Ferner können solche Membranen optisch in Reflexion oder Transmission analytisch untersucht werden. Hauptanwendungsgebiete dieser analytischen Meßmethode mit Hilfe von Membranen liegen auf dem Gebiet der medizinischen Diagnostik, aber auch in der Umweltanalytik.

Bei Biosensoren, insbesondere solchen auf der Basis elektrochemischer Transducer, ist es ein technologisch bislang nur unzureichend gelöstes Problem, auf den eigentlichen Transducer die biologische(n) Komponente(n) aufzubringen, die als Indikator (Antikörper, Antigene, DNA, RNA) oder Katalysator (Enzyme) benutzt werden, Diese Moleküle sind im allgemeinen empfindlich gegenüber organischen Lösungsmitteln. Bei Kontakt mit organischen Lösungsmitteln muß mindestens mit einer partiellen Denaturierung der Biopolymere gerechnet werden. Es ist notwendig, daß die Immobilisierung unter Ausschluß nichtwäßriger Lösungsmittel erfolgt. Die meist verwendete Technik ist eine kovalente Immobilisierung über Seitengruppen des Biopolymers mittels einer radikalischen Reaktion. Dabei erfolgt Anbindung an ein bereits vorliegendes Polymer mit z.B. Aldehyd-Seitengruppen. Eine andere Möglichkeit ist die Erzeugung eines Polymers in situ (z.B. Polyacrylamidgele), was in einem physikalischen Einschluß der Makromoleküle resultiert. Radikalische Immobilisierungsreaktionen sind im allgemeinen mit einem hohen Verlust an biologischer Aktivität verbunden, die durch einen höheren kostenintensiven, Materialeinsatz kompensiert werden muß.

Halbleitertransducer für Biosensoren, wie z.B. ISFETs, sind zudem häufig instabil gegenüber radikalischen Immobilisierungsreaktionen auf ihren "Gate"-zonen. Dies führt zu hohen Ausfallraten, da die radikalischen Agentien auch mit den dünnen Schichten z.B. der "Gate"-isolatoren reagieren und deren Struktur angreifen.

Für die Immobilisierung von Enzymen kommen heute zum Einsatz:
1. Die kovalente Anbindung an aktivierte wasserunlösliche Trägermaterialien. Diese sind meist Polysaccharide, Silikate oder leitfähige Polymere.
2. Die Einbettung in eine Matrix, z.B. Polyacrylamidgele,
3. die Adsorption an Oberflächen.

Eine Ubersicht der klassischen Immobilisierungstechniken für Enzyme und andere Biopolymere findet sich u.a. bei P.W. Carr, L.D. Bowers "Immobilized Enzymes in Analytical and Clinical Chemistry" in "Chemical Analysis", Vol. 56 (eds. P.J. Elving, J.D. Winefordner), J. Wiley & Sons, New York.

Für chemische Sensoren bzw. Biosensoren sind zwei spezielle Varianten beschrieben. Zum einen die Immobilisierung von Enzymen mittels bzw. an leitfähigen Polymeren wie z.B. Polypyrrol (N.C. Foulds, C.R. Lowe "Enzyme Entrapment in Electrically Conducting Polymers", J. Chem. Soc., Faraday Trans. 1, 82 (1986) 1259-1264) sowie die Verwendung von Kohleschlämmen bzw. Pasten, die mit Enzymen gemischt werden und z.B. mittels Siebdruck auf Oberflächen aufgebracht werden (EP 0 351 891 A2).

In Abhängigkeit der verwendeten Immobilisierungsmethode muß das Verfahren zur Applikation der Biopolymer-haltigen Schichten auf den Substraten gewählt werden. Meist kann für die Aufbringung der biologischen Komponenten auf die Transducer und die eigentliche Transducer-Herstellung nicht die gleiche Technik bzw. das gleiche Verfahren (z. B. der Siebdruck) verwendet werden.

Eine Übersicht der geläufigen Transducer und deren Konstruktion und Gestaltung findet sich in:
J. Janata, A. Bezegh, "Chemical Sensors", Anal. Chem. 60 (1988) 62R-74R. C. Nylander, "Chemical and Biological Sensors", J. Phys. E. Sci. Instrum. 18 (1985) 736-750.

Der Erfindung liegt die Aufgabe zugrunde, die Immobilisierung von organischen Makromolekülen oder Biopolymeren in einer Polymermembran hinsichtlich der Lebensdauer und der Erhaltung der biologischen Aktivität zu verbessern. Der auf ein Festkörpersubstrat, z. B. Papier, Keramik, Metall, Glas oder Kunststoff, aufgebrachte Polymerfilm mit dem immobilisierten Biopolymer soll trocken lagerfähig sein und nach Rehydratisierung eine hohe biologische Aktivität aufweisen. Für die Anwendung solcher Membranen bei der Herstellung von Biosensoren ist es ferner wesentlich, daß die Membranen hinsichtlich der Meßempfindlichkeit eine hohe Reproduzierbarkeit aufweisen und bei der Herstellung der Membranen mit den gleichen Maschinen und Vorrichtungen gearbeitet werden kann wie bei der Herstellung der Transducer für die Biosensoren.

Diese Aufgabe wird - ausgehend von einem Immobilisierungsverfahren, bei dem die Makromoleküle oder Biopolymere mit einer wäßrigen, lösungsmittelfreien, nicht ionogenen Polymerdispersion vermischt werden und diese Mischung in Form eines zusammenhängenden Films aufgetragen und getrocknet wird, - erfindungsgemäß dadurch gelöst, daß eine polydisperse Polymerdispersion verwendet wird, die einen Anteil von 60 % bis 99 %, vorzugsweise 90 % bis 97 %, an Teilchen mit einem Durchmesser von 0,1 µm bis 1 µm aufweist und deren restlicher Anteil einen Teilchendurchmesser von 1 µm bis 10 µm besitzt. Vorzugsweise liegt dabei der Durchmesser des Hauptanteils der Teilchen im Bereich von 0,3 µm bis 0,8 µm, während der Durchmesser des Restanteils im Bereich von 2 bis 5 µm liegt.

Ziel der Immobilisierung ist also eine hohe Biopolymermenge, z. B. ein Enzym, mit hoher Aktivitätsausbeute in einer Membran zu fixieren. Erwünscht ist dabei die Aufnahmefähigkeit der Membran für Wasser, um die Enzyme in aktiver Form zu erhalten, die Durchlässigkeit für Enzymsubstrate und gegebenenfalls der Ausschluß von bestimmten Komponenten, wie z. B. roten Blutkörperchen. "Nicht ionogen" heißt, daß außer den grundsätzlich vorhandenen Polymerseiten- und Endgruppen keine anionischen oder kationischen Gruppen vorhanden sind. Dies bedeutet, daß mit global ungeladenen Polymeren gearbeitet wird. lonogene Gruppen können zu empfindlichen Störungen führen, wenn die Polymermembran bei elektrochemischen Meßverfahren eingesetzt wird.

Es wurde gefunden, daß die kleinen Teilchen (mit einem Durchmesser zwischen 0,1 µm und 1,0 µm) aufgrund ihrer großen Gesamtoberfläche für die Absorption der Biopolymeren bzw. für deren Matrixeinschluß von wesentlicher Bedeutung sind. Sie sind auch für die eigentliche Filmbildung und die Filmhaftung und damit für die Biopolymeren-Immobilisierung verantwortlich. Dagegen dienen die Anteile mit größeren Teilchendurchmessern, d. h. 1 µm bis 10 µm (vorzugsweise 2 µm bis 5 µm), der Strukturbildung des Films, bestimmen die Wasseraufnahmefähigkeit und sind maßgeblich verantwortlich für die durchschnittliche Porengrößenverteilung der Filme, welche wiederum für die Substratpermeabilität z. B. für immobilisierte Enzyme wichtig ist.

Durch die Einstellung des Verhältnisses der Teilchenkonzentrationen in den beiden Größenklassen lassen sich die sensorrelevanten Eigenschaften (Substratpermeabilität), Hydrathöhlen für Biopolymere, Immobilisierungskapazität etc.) variieren und anpassen.

Eine weitere Verbesserung besteht darin, daß der Polymerdispersion innere und/oder äußere Weichmacher zugesetzt werden. Innere Weichmacher, die z. B. durch Copolymerisation mit Ethylen in Polymere eingeführt werden können, erhöhen die Flexibilität des Filmes und wirken sich positiv auf die Dauerhaftfähigkeit auch bei Temperaturschwankungen aus.

Äußere Weichmacher, z.B. Dibutylphthalat = DBP, bringen zwar die Gefahr der Ausdiffusion mit sich, haben sich jedoch insbesondere bei "Sandwich-Membranen", z.B. bei einer darunterliegenden potentiometrischen auf einer PVC-Matrix basierenden Membran, die ebenfalls mit DBP weich gemacht ist, als vorteilhaft erwiesen.

Durch Kombination innerer und äußerer Weichmacher können die Membraneigenschaften im Hinblick auf die Laminierungsfähigkeit mit anderen Membranen/-typen und die Kälte- und Wärmeausdehnungseigenschaften (Dauerflexibilität) und damit die Haftfähigkeit auf Oberflächen mit abweichenden Wärmeausdehnungskoeffizienten, z.B. Keramik, Epoxide, Papier etc., verbessert werden.

Oberflächenaktive Substanzen haben durch ihren Tensidcharakter im allgemeinen eine schädliche Wirkung auf Biopolymere, da sie häufig zur Denaturierung und damit zum Aktivitätsverlust führen.

Polyvinylalkohol hat sich als ein sehr gut enzymverträgliches, zur Stabilisierung der Polymerdispersion beitragendes Schutzkolloid erwiesen.

Nach der Trocknung kann der Polymerfilm vom Substrat abgezogen werden und bildet dann eine geschlossene zusammenhängende Membran mit einer auf die Fläche bezogenen, konstanten Porosität. Auf diese Weise können Membranen mit hoher Dehnbarkeit (ca. 550 %) und Reißfestigkeit (ca. 4 N/mm ) hergestellt werden. Für die meisten Anwendungen wird die Membran jedoch nicht abgelöst, sondern bleibt auf dem Substrat. Insbesondere kann die Meßelektrode eines Transducers für einen Biosensor, der in diesem Fall das Substrat darstellt, nach dem erfindungsgemäßen Verfahren mit einer Polymermembran beschichtet werden. Geeignete Transducer für Biosensoren können mit Hilfe von Dickschicht- oder Dünnschichtschaltungen hergestellt werden und mit Halbleiterbausteinen, wie Feldeffekttransistoren und deren Modifikationen (ISFET, ChemFET) kombiniert werden.

Eine vorteilhafte Methode zur reproduzierbaren Beschichtung des Substrats besteht darin, daß eine vorgegebene Menge der Biopolymer-/Polymerdispersionsmischung zeitgesteuert mittels Druckluft aus einer Kartusche auf die Substratoberfläche dosiert wird. Zur Durchführung dieses Verfahrens können die in der elektronischen Fertigungstechnik üblichen SMD-Dosierer benutzt werden. Durch die exakte Positionierung der Austrittsöffnung der Dosiereinrichtung kann die Auftragsstelle auf dem Substrat mit hoher Präzision festgelegt werden. Die Reproduzierbarkeit der Dosiermenge ist typischerweise 1 %. Ein wesentlicher Vorteil dieses Verfahrens besteht darin, daß durch die geringe Fläche der Dosierspitze ein Verlust an Wasser praktisch ausgeschlossen ist und die Zusammensetzung der Biopolymer-/Polymerdispersionsmischung sich auch während längerer Verarbeitungsperioden nicht ändert. Außerdem ermöglicht diese Technik praktisch eine vollständige Verarbeitung der aufgrund des Biopolymergehalts gegebenenfalls sehr wertvollen Mischung. Die Flexibilität dieses Verfahrens ermöglicht ferner die Beschichtung eines Transducers mit verschiedenen Membranen in einem einzigen Arbeitsgang. Die Verwendung von Mehrfachdosierern (z.B. mit Revolverhalterung) erlaubt es nach einmaliger Positionierung des Transducers, alle Elektrodenpunkte mit den unterschiedlichen Membranen in einem Arbeitsgang zu beschichten.

Durch Zusatz von lipophilen Farbstoffen zu der Biopolymer-/Polymerdispersionsmischung kann man erreichen, daß die Membran lichtundurchlässig wird, wodurch störende Photoeffekte an den Elektrodenoberflächen eliminiert werden können. Des weiteren kann durch Zusatz von Metall- oder Graphitstaub die elektrische Leitfähigkeit der Membran heraufgesetzt werden. Die elektrische Leitfähigkeit ist von Bedeutung, wenn Ladungsträger, die z.B. aus Redox-Prozessen stammen, abgeführt werden müssen.

Gemäß einer Weiterentwicklung der Erfindung wird eine laminierte Schichtfolge von Membranen mit hoher wechselseitiger Adhäsion durch sukzessives Auftragen der lösungsmittelfreien Polymerdispersion hergestellt, wobei mindestens eine Schicht mit dem Zusatz von Makromolekülen bzw. Biopolymeren versehen ist. Die Herstellung derartiger laminierter Membranschichten erfolgt zweckmäßig mit der oben beschriebenen SMD-Dosiertechnik oder durch Siebdruck, Anwendungsbeispiele für Biosensoren mit laminierten Membranschichten werden weiter unten beschrieben.

Die erfindungsgemäß hergestellten biopolymerhaltigen Membranen weisen folgende vorteilhafte Eigenschaften auf: Sie sind
- dauerelastisch; d.h. sie bleiben auch bei mechanischer Beanspruchung in innigem Kontakt mit dem Substrat
- trockenlagerfähig; d.h. während der Lagerung tritt kein Verlust an Enzymaktivität auf
- dauerhaftfähig; d.h. sie sind kaum oder nur schwer vom Substrat abzulösen
- wasserunlöslich; d.h. sie zeigen bei dem Kontakt mit dem Analyten keinerlei Auflösungserscheinungen, was einen Dauereinsatz verbieten würde
- wasseraufnahmefähig; d.h. eine Rehydratisierung der immobilisierten Biopolymere ist gewährleistet und
- in reproduzierbarer Weise porös, d.h. für den Durchtritt kleiner Moleküle, wie z.B. Enzymsubstrate, geeignet, aber andererseits undurchlässig für makroskopische Komponenten, z.B. rote Blutkörperchen oder Feststoffanteile im Analyten.

Ein weiterer wichtiger Vorteil liegt ferner darin, daß die Beschichtung der Transducer mit der gleichen Technik und den gleichen Einrichtungen erfolgen kann wie die Herstellung der biopolymerhaltigen Membran. Auf diese Weise kann die Herstellung des kompletten Biosensors vereinfacht werden, was zu einer Kostenreduktion führt und ein Konzept für einen "Einweg-Sensor" für einen einmaligen Gebrauch eröffnet.

Im folgenden wird die Erfindung anhand von Zeichnungen und Ausführungsbeispielen näher erläutert.

Es zeigen
- Fig. 1 und Fig. 2: die Beschichtung einer Meßelektrode in einem Biosensor mit einer Biopolymer-/Polymerdispersion
- Fig. 3: ein aus zwei Schichten bestehendes Polymermembranlaminat für einen Glukose-Sensor
- Fig. 4: die prinzipielle Meßfeldanordnung bei einem Dickschicht-Hybrid-Sensor und
- Fig. 5: ein nach dem Totalreflexionsprinzip arbeitender optischer Sensor mit einer Biopolymer-Polymerschicht.

Bei der Auswahl einer geeigneten Polymerdispersion zur Immobilisierung von Biopolymeren in einer Membran spielen folgende Gesichtspunkte eine Rolle:
1. Während der Filmbildung dürfen keine chemischen Reaktionen, insbesondere keine Quervernetzung stattfinden, da sonst Aktivitätsverluste durch kovalente Enzymimmobilisierung eintreten würden.
2. Eine polydisperse Partikelgrößencharakteristik bedingt auch eine inhomogene Porenweitenverteilung innerhalb des ganzen Polymers.
3. Die Polymerdispersion darf keine Monomere enthalten (Unverträglichkeit mit Enzymaktivität).
4. Die Filme bzw. Membranen lassen sich mit sehr guter Reproduzierbarkeit herstellen, da als Verfahrensparameter nur die Luftfeuchtigkeit und die Temperatur zu beachten sind und keine chemischen Reaktionsparameter eingestellt bzw. kontrolliert werden müssen.
5. Die Polymere sollen elektrisch ungeladen sein, d.h. keine ionogenen Gruppen enthalten, die bei einer Verwendung der Membran in einem elektrochemischen Sensor zu Interaktionen und Störungen führen können.
6. Die Lagerfähigkeit bleibt auch im tiefgefrorenen Zustand erhalten. Die Filme werden nicht spröde sondern bleiben dauerelastisch.

Für die nachfolgenden Beispiele wurde eine lösungsmittelfreie wäßrige Vinnapas®-Dispersion M54/25C der Firma Wacker-Chemie verwandt. Es handelt sich dabei um eine äußerlich weichgemachte homopolymere Polyvinylacetat-Dispersion mit Dibutylphthalat als Weichmacher und Teiichengrößen im Bereich von 0,5 bis 2 µm. Zur Herstellung der Biopolymer-/Polymermischung werden die in einer Pufferlösung befindlichen Enzyme mit der Vinnapas®-Dispersion vermischt. Diese Enzym-/Polymermischung ist lagerfähig.

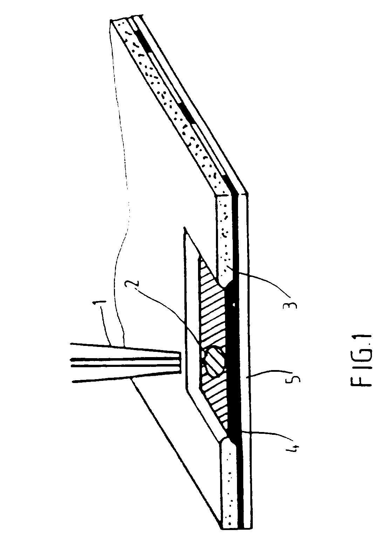

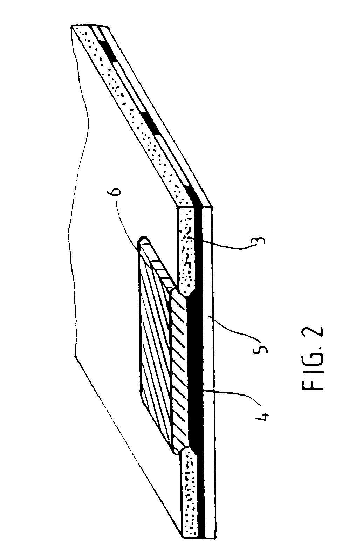

Gemäß Fig. 1 wird mit Hilfe einer Mikrodosiereinrichtung 1 ein Tropfen 2 der Enzym-/Polymermischung auf die mit einer Passivierungsschicht 3 abgegrenzte Meßelektrode 4 eines Biosensors aufgebracht. Die Meßelektrode 4 ist auf einem Keramikträger 5 angeordnet. Ein Biosensor dieser Bauart ist ausführlich in DE 3 827 314 beschrieben. Nach der Benetzung der Meßelektrode 4, die durch Klopfen bzw. Vibration unterstützt werden kann, bildet sich ein gleichmäßiger Polymerfilm auf der Oberfläche aus, der bei Zimmertemperatur ca. 5 bis 20 min getrocknet wird und dann eine gleichmäßige Membran 6 (siehe Fig. 2) bildet, in der die Enzyme immobilisiert sind.

Alternativ kann die Enzym-/Polymerdispersionsmischung auch mittels Siebdruck appliziert werden. Die getrocknete Polymerschicht 6 bildet einen wasserunlöslichen Film, der ausreichend porös ist, um den Zutritt von Molekülen, z.B. Glukose, an die immobilisierten biologischen Komponenten zu gestatten, jedoch einen Ausschluß von z.B. roten Blutkörperchen gewährleistet. Die Trockenlagerfähigkeit dieser Enzymmembranen ist sehr gut. Durch geeignete Wahl der Seitenketten der Polymere kann ein lokales Puffersystem erzeugt werden, welches für die enzymatischen Reaktionen vorteilhaft ist. Eine höhere Trockentemperatur kann gewählt werden, wenn die Biokompatibilität des Enzyms gewährleistet bleibt; d.h. wenn sichergestellt ist, daß keine Schädigung der biologischen Aktivität während der Trocknung eintritt.

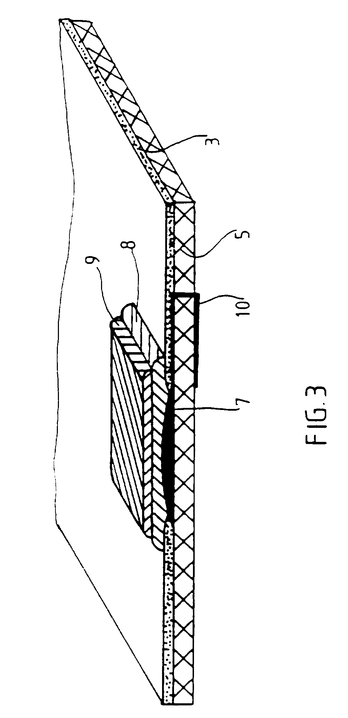

Gemäß Fig. 3 wird eine die Elektrodenzone 7 abdeckende Doppelschichtmembran (Laminat aus Schichten 8 und 9) dadurch hergestellt, daß auf die Oberfläche einer ersten, bereits getrockneten Membranschicht 8 eine zweite Membranschicht 9 aufgetragen wird. Die Elektrodenzone 7 befindet sich wiederum auf einem Keramiksubstrat 5 und ist nach außen hin durchkontaktiert (Anschluß 10), Nachfolgend werden drei Anwendungsbeispiele für derart aufeinander laminierte Membranschichten bei Biosensoren beschrieben.

### 1. Glukose-Sensor mit größenselektiver Schutzschicht zur Abtrennung von Serum aus Vollblut

Über eine Glukose-Oxidase (GOD)-haltige Membran 8 wird eine zweite enzymfreie Membran 9 mit größerer Porosität laminiert. Durch die obere Membran 9 wird eine Abtrennung von Serum aus Vollblut erzielt und die Verfälschung der Glukose-Bestimmung durch unspezifische Effekte minimiert.

### 2. Glukose-Sensor mit erhöhtem Linearitätsbereich

Über einen analog aufgebauten Glukcse-Sensor wird eine zweite Membran 9 mit dem Enzym Katalase laminiert. Freigesetztes H₂O₂, das bei der GOD-Reaktion entsteht und nicht an der Elektrode umgesetzt wird, wird in der Katalase-Schicht zu O₂ umgesetzt. Dadurch wird der Linearitätsbereich des Glukose-Sensors in Richtung höherer Glukose-konzentrationen erweitert.

### 3. Harnstoff-Sensor

Auf eine konventionelle Ammonium-selektive PVC-Membran 8 wird eine Urease-haltige Membran 9 auflaminiert. Dadurch entsteht ein Harnstoffsensor.

In allen Fällen sind die Membranschichten 8 und 9 klar voneinander separiert, haften aber andererseits fest aneinander (hohe Adhäsion). Die hier beschriebenen Schichtstrukturen sind wiederum als Hauptbestandteil des in DE 3 827 314 beschriebenen Biosensors anzusehen.

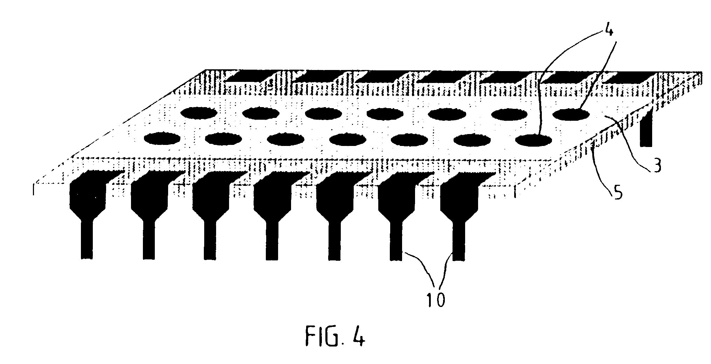

Bei dem in Fig. 4 dargestellten Dickschicht-Hybrid-Sensor sind eine Vielzahl von Meßelektroden-Feldern 4 auf einem Keramik-Träger 5 aufgebracht. Analog zu der Anordnung gemäß Fig. 1 sind die Meßfelder 4 in eine Isolatorschicht (Passivierungsschicht) 3 eingebettet. Die Meßfelder (Meßelektroden) 4 werden, wie oben beschrieben, mit einer Enzym-/Polymerdispersion beschichtet und sind mit individuellen Kontaktanschlußfahnen 10 versehen.

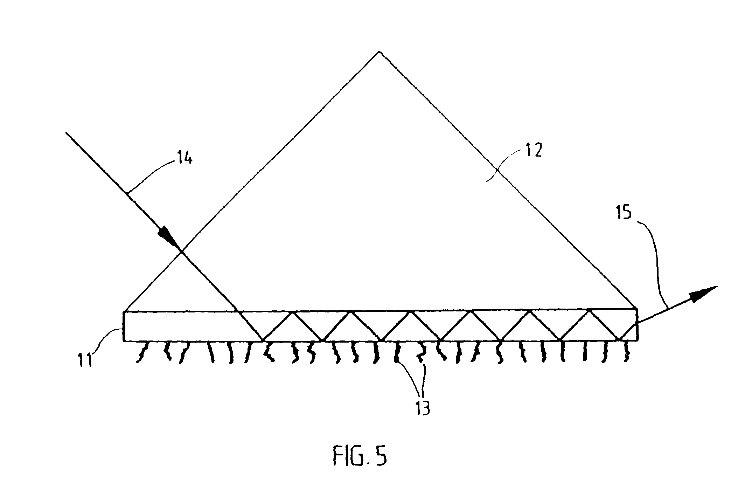

Eine gänzlich andere meßtechnische Anwendung ist in Fig. 5 dargestellt. Die Anordnung arbeitet als optischer Sensor nach dem Totalreflexionsprinzip. Die Enzym-/Polymerdispersionsschicht 11 wird hier auf die Unterseite eines Glasprismas 12 aufgebracht. Das an der Meßoberfläche wirksame immobilisierte Enzym ist mit 13 bezeichnet. Das Sensorprinzip beruht darauf, daß die Intensität eines vielfach an der Meßoberfläche total reflektierten Lichtstrahls 14 in charakteristischer Weise durch Enzym-Reaktionen in dem zu untersuchenden, an die Meßoberfläche angrenzenden Medium verändert wird. Als Meßwert dient dabei in bekannter Weise das Intensitätsverhältnis von austretendem Lichtstrahl 15 und eintretendem Lichtstrahl 14.

## Patentansprüche

1. Verfahren zur Immobilisierung von organischen Makromolekülen oder Biopolymeren, insbesondere von Enzymen, Antikörpem, Antigenen, DNA/RNA und von Mikroorganismen, bei dem die zu immobilisierenden Makromoleküle oder Biopolymere mit einer wäßrigen, lösungsmittelfreien, nicht ionogenen Polymerdispersion vermischt werden und diese Mischung in Form eines zusammenhängenden Films auf ein Substrat aufgetragen wird, der anschließend getrocknet wird, dadurch gekennzeichnet, daß eine polydisperse Polymerdispersion verwendet wird, die einen Anteil von 60 % bis 99 %, vorzugsweise 90 % bis 97 %, an Teilchen mit einem Durchmesser von 0,1 µm bis 1 µm aufweist und deren restlicher Anteil einen Teilchendurchmesser von 1 µm bis 10 µm besitzt.

2. Verfahren nach Anspruch 1, dadurch gekennzeichnet, daß der Hauptanteil der Teilchen einen Durchmesser von 0,3 µm bis 0,8 µm und der Restanteil der Teilchen einen Durchmesser von 2 µm bis 5 µm aufweist.

3. Verfahren nach den Ansprüchen 1 und 2, dadurch gekennzeichnet, daß die Polymerdispersion innere und/oder äußere, vorzugsweise mit einem Enzym verträglichen Schutzkolloid versehene Weichmacher enthält.

4. Verfahren nach den Ansprüchen 1 bis 3, dadurch gekennzeichnet, daß als Substrat die Meßelektrode eines Transducers für einen Biosensor verwendet wird.

5. Verfahren nach den Ansprüchen 1 bis 4, dadurch gekennzeichnet, daß zur reproduzierbaren Beschichtung des Substrats eine vorgegebene Menge der Biopolymer/Polymer-Dispersionsmischung zeitgesteuert mittels Druckluft aus einer Kartusche auf die Substratoberfläche dosiert wird.

6. Verfahren nach den Ansprüchen 1 bis 5, dadurch gekennzeichnet, daß der Biopolymer/Polymer-Mischung lipophile Farbstoffe zugesetzt werden.

7. Verfahren nach den Ansprüchen 1 bis 6, dadurch gekennzeichnet, daß die Biopolymer/Polymer-Mischung durch Zusätze von Metall- oder Graphitstaub leiffähig gemacht wird.

8. Verfahren nach den Ansprüchen 1 bis 7, dadurch gekennzeichnet, daß eine laminierte Schichtfolge von Membranen mit hoher wechselseitiger Adhäsion durch sukzessives Auftragen einer lösungsmittelfreien Polymerdispersion hergestellt wird, wobei mindestens eine Schicht mit dem Zusatz von Makromolekülen bzw. Biopolymeren versehen ist.

## Claims

1. Method for immobilising organic macromolecules or biopolymers, in particular enzymes, antibodies, antigens, DNA/RNA and microorganisms, whereby the macromolecules or biopolymers to be immobilised are mixed with an aqueous, solvent-free, nonionic polymer dispersion and this mixture is applied in the form of a coherent film to a substrate, which is subsequently dried, characterised in that a polydisperse polymer dispersion is used, which dispersion contains a proportion of from 60% to 99%, preferably of from 90% to 97%, of particles having a diameter of from 0.1 µm to 1 µm and the remaining proportion thereof has a particle diameter of from 1 µm to 10 µm.

2. Method according to claim 1, characterised in that the major proportion of the particles has a diameter of from 0.3 µm to 0.8 µm and the remaining proportion of the particles has a diameter of from 2 µm to 5 µm.

3. Method according to claims 1 and 2, characterised in that the polymer dispersion contains internal and/or external plasticisers, preferably provided with a protective colloid compatible with an enzyme.

4. Method according to claims 1 to 3, characterised in that the substrate used is the measuring electrode of a transducer for a biosensor.

5. Method according to claims 1 to 4, characterised in that, for the reproducible coating of the substrate, a given quantity of the biopolymer/polymer dispersion mixture, timed by means of compressed air from a cartridge, is measured out onto the surface of the substrate.

6. Method according to claims 1 to 5, characterised in that lipophilic dyes are added to the biopolymer/ polymer mixture.

7. Method according to claims 1 to 6, characterised in that the biopolymer/polymer mixture is rendered conductive by additions of metal dust or graphite dust.

8. Method according to claims 1 to 7, characterised in that a laminated series of layers of membranes having high mutual adhesion is prepared by gradual application of a solvent-free polymer dispersion, with at least one layer being supplied with the addition of macromolecules or biopolymers.

## Revendications

1. Procédé pour immobiliser des macromolécules ou des biopolymères organiques, notamment des enzymes, des anticorps, des antigènes, de l'ADN/ARN et des microorganismes, dans lequel les macromolécules ou les biopolymères à immobiliser sont mélangés avec une dispersion aqueuse de polymère non ionogène dépourvue de solvant et ce mélange est appliqué sous forme d'un film cohérent sur un substrat qui est ensuite séché, caractérisé en ce qu'on utilise une dispersion de polymère polydispersé qui présente une proportion de 60 % à 99 %, de préférence de 90 % à 97 %, de particules ayant un diamètre de 0,1 µm à 1 µm et dont la portion restante possède un diamètre de particules de 1 µm à 10 µm.

2. Procédé suivant la revendication 1, caractérisé en ce que la proportion dominante des particules présente un diamètre de 0,3 µm à 0,8 µm et la proportion restante des particules présente un diamètre de 2 µm à 5 µm.

3. Procédé suivant les revendications 1 et 2, caractérisé en ce que la dispersion de polymère contient des plastifiants internes et/ou externes pourvus de préférence d'un colloïde protecteur compatible avec un enzyme.

4. Procédé suivant les revendications 1 à 3, caractérisé en ce qu'on utilise comme substrat l'électrode de mesure du transducteur d'un capteur biologique.

5. Procédé suivant les revendications 1 à 4, caractérisé en ce que, pour le revêtement reproductible du substrat, on fait arriver à sa surface depuis une cartouche au moyen d'air comprimé, d'une manière réglée dans le temps, une quantité prédéterminée du mélange en dispersion biopolymère/polymère.

6. Procédé suivant les revendications 1 à 5, caractérisé en ce que des colorants lipophiles sont ajoutés au mélange biopolymère/polymère.

7. Procédé suivant les revendications 1 à 6, caractérisé en ce que le mélange biopolymère/polymère est rendu conducteur par des additions de métal ou de graphite en poudre.

8. Procédé suivant les revendications 1 à 7, caractérisé en ce qu'on produit une succession de couches stratifiées de membranes ayant une haute adhésion mutuelle par l'application successive d'une dispersion de polymère sans solvant, au moins une couche étant pourvue de l'adjonction de macromolécules ou de biopolymères.
